(19) Europäisches Patentamt
European Patent Office
Office européen des brevets

(11) **EP 3 909 445 A1**

(12) **EUROPEAN PATENT APPLICATION**
published in accordance with Art. 153(4) EPC

(43) Date of publication:
**17.11.2021 Bulletin 2021/46**

(51) Int Cl.:
*A24F 47/00* (2020.01)   *C12Q 1/06* (2006.01)
*C12Q 1/6837* (2018.01)   *C12Q 1/6851* (2018.01)
*C12Q 1/686* (2018.01)   *G01N 33/50* (2006.01)

(21) Application number: **19909136.4**

(22) Date of filing: **16.12.2019**

(86) International application number:
**PCT/JP2019/049086**

(87) International publication number:
**WO 2020/145041 (16.07.2020 Gazette 2020/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.01.2019   JP 2019003076**

(71) Applicant: **Japan Tobacco Inc.**
**Tokyo 105-6927 (JP)**

(72) Inventor: **MATSUMURA, Kazushi**
**Tokyo 130-8603 (JP)**

(74) Representative: **Hoffmann Eitle**
**Patent- und Rechtsanwälte PartmbB**
**Arabellastraße 30**
**81925 München (DE)**

(54) **IN VITRO EVALUATION METHOD FOR RISK OF CHRONIC OBSTRUCTIVE PULMONARY DISEASE ASSOCIATED WITH SMOKING OR INHALATION**

(57) The present invention relates to an in vitro method for evaluating the risk of chronic obstructive pulmonary disease associated with smoking or inhalation. The method of the present invention comprises: (1) calculating probability $P_{SMK}$ on the basis of data on the expression of 11 or more genes including at least 11 specific genes from among the following 15 genes in airway epithelial cells exposed to a sample solution containing an aerosol or particulate matter in the aerosol from a tobacco product: TXNIP, EFNA1, CYP1B1, ADM, AREG, DUSP6, SLC7A11, IGFBP3, WNT5A, CXCR4, PHLDA1, HILPDA, ZBED2, EGLN3, and FBXO32, with use of an intercept and coefficients calculated in advance by logistic regression analysis using public data; (2) calculating probability $P_{COPD}$ on the basis of data on the expression of 4 or more genes including at least 4 specific genes from among the following 15 genes in airway epithelial cells exposed to a sample solution containing the an aerosol or particulate matter in the aerosol from a tobacco product, with use of coefficients calculated in advance by logistic regression analysis using public data; and (3) calculating the risk of chronic obstructive pulmonary disease associated with smoking or inhalation of the aerosol or particulate matter in the aerosol from the tobacco product from the probability $P_{SMK}$ and the probability $P_{COPD}$.

Fig. 5

**Description**

TECHNICAL FIELD

The present invention relates to an in vitro method for evaluating the risk of chronic obstructive pulmonary disease associated with smoking or inhalation, and a heated tobacco product.

BACKGROUND ART

Chronic Obstructive Pulmonary Disease (as used herein, hereinafter sometimes referred to as "COPD") is an inflammatory respiratory disease that occurs due to long-term inhalation exposure to harmful substances such as air pollutants, dust, and microorganisms. Since tobacco smoke is considered to be one of the major risk factors for COPD, there is an urgent need to establish a technique for evaluating the risk of tobacco smoke to develop COPD. In this regard, animal models for smoking exposure studies have also been reported, but there are concerns about differences between species when the effects on humans are extrapolated from the results of evaluation on the animal models.

Under these circumstances, the importance of predicting disease risk to humans by in vitro tests using human-derived cells is increasing in the tobacco industry. In particular, since an airway epithelial tissue is a tissue that acts as the first defense mechanism against inhaled harmful substances, the biological effects of tobacco smoke are being actively evaluated using airway epithelial cells. In recent years, in addition to conventional single exposure tests on monolayer cultured cells, evaluation on a test system with high extrapolation to humans, such as a frequent exposure test on a three-dimensional culture model having a tissue structure closer to that of a living body, has also been aggressively carried out.

In recent years, non-traditional tobacco products such as electronic cigarettes and heated tobacco products have been developed and put on the market. These products are characterized by the fact that they produce aerosols without burning, and thus are considered to be able to suppress the generation of chemical substances that are generated by burning and the chemical reaction of the components in the chopped tobacco with the high-temperature combustion gas, as found in conventional combustible cigarettes. From the results of component analysis of these tobacco products, it has been actually reported that some of the biologically active chemical components therein are quantitatively lower than those of combustible cigarettes.

Use of various omics technologies is attracting attention as a technique for comprehensively analyzing complex biological responses to chemical substances contained in smoke and an aerosol from smoking articles. In particular, measurement of gene expression using qPCR arrays and microarrays has extremely higher data quality compared to other omics technologies due to major technological advances, and the number of registrations in public databases is abundant. Therefore, meta-analysis using array data information is also actively performed. It has been reported that changes in gene expression in airway epithelial cells are suppressed in exposure to the smoke of electronic cigarettes or heated tobacco products having low contents of biologically active chemical substances than in exposure to combustible cigarette smoke. However, in the current situation, any specific evaluation technique has not yet been established regarding a technique for predicting a specific disease risk from fluctuations in gene expression levels caused by these biologically active chemical substances.

WO2013/190092 describes a group of genes to be used for the risk of COPD. Am. J. Respir Crit Care Med. 2007, 175 (6): 577-586 reports genes characteristic of COPD patients. However, no effective method has yet been provided for statistically quantitatively evaluating the risk of chronic obstructive pulmonary disease associated with smoking or inhalation.

CITATION LIST

PATENT LITERATURE

PTL 1: WO2013/190092
PTL 2: WO2016/075748
PTL 3: WO2016/075749

NON PATENT LITERATURE

NPL 1: Am. J. Respir Crit Care Med. 2007, 175 (6): 577-586
NPL 2: ISO 3308

SUMMARY OF INVENTION

TECHNICAL PROBLEM

An object of the present invention is to provide an in vitro method for evaluating the risk of chronic obstructive pulmonary disease associated with smoking or inhalation, and a heated tobacco product.

SOLUTION TO PROBLEM

The present inventors have newly constructed a technique for evaluating tobacco products for potential COPD risk based on an in vitro evaluation system using microarray data, and have evaluated the biological effects of exposure of a three-dimensional cultured cell model to an aerosol from heated tobacco. As a result, the present inventors have found that the potential COPD risk of the heated tobacco product is low, and have conceived the present invention.
Without limitation, the present invention includes the following embodiments.

[Embodiment 1] An in vitro method for evaluating the risk of chronic obstructive pulmonary disease associated with inhalation, comprising:

(1) calculating the following probability $P_{SMK}$ on the basis of data on the expression of 11 or more genes including at least the following 11 genes:
TXNIP, EFNA1, ADM, SLC7A11, IGFBP3, WNT5A, HILPDA, EGLN3, CXCR4, AREG, and FBXO32 from among the following 15 genes in airway epithelial cells exposed to a sample solution containing an aerosol or particulate matter in the aerosol from a heated tobacco product:
TXNIP, EFNA1, CYP1B1, ADM, AREG, DUSP6, SLC7A11, IGFBP3, WNT5A, CXCR4, PHLDA1, HILPDA, ZBED2, EGLN3, and FBXO32,
with use of an intercept and coefficients calculated in advance by logistic regression analysis using public data;

[Chemical Formula 1]

$$logit(P_{SMK}) = ln\left(\frac{P_{SMK}}{1 - P_{SMK}}\right) = C_{NS/SMK} + \beta_1 a_1 + \beta_2 a_2 + \cdots + \beta_x a_x$$

wherein $C_{NS|SMK}$ represents the intercept, $\beta_i$ represents the coefficient for each gene, $a_i$ represents gene expression data and represents a value after normalization of microarray data by GC-RMA or a quantitative value of gene expression obtained by a qPCR method, and x represents any integer of 11 to 15;
(2) calculating the following probability $P_{COPD}$ on the basis of data on the expression of 4 or more genes including at least the following 4 genes:
EFNA1, TXNIP, DUSP6, and AREG
from among the following 15 genes in airway epithelial cells exposed to a sample solution containing an aerosol or particulate matter in the aerosol from a heated tobacco product:
TXNIP, EFNA1, CYP1B1, ADM, AREG, DUSP6, SLC7A11, IGFBP3, WNT5A, CXCR4, PHLDA1, HILPDA, ZBED2, EGLN3, and FBXO32,
with use of an intercept and coefficients calculated in advance by logistic regression analysis using public data;

[Chemical Formula 2]

$$logit(P_{COPD}) = ln\left(\frac{P_{COPD}}{1 - P_{COPD}}\right) = C_{SMK/COPD} + \gamma_1 b_1 + \gamma_2 b_2 + \cdots + \gamma_y b_y$$

wherein $C_{SMK|COPD}$ represents the intercept, $\gamma_i$ represents the coefficient for each gene, $b_i$ represents gene expression data and represents a value after normalization of microarray data by GC-RMA or a quantitative value of gene expression obtained by the qPCR method, and y represents any integer of 4 to 10; and

(3) calculating the risk of chronic obstructive pulmonary disease associated with inhalation of the heated tobacco product, PRF, from the probability $P_{SMK}$ and the probability $P_{COPD}$.

[Chemical Formula 3]

$$PRF = \frac{p_{SMK} \times p_{COPD}}{1 - (p_{SMK} \times p_{COPD})}$$

[Embodiment 2] The in vitro method according to Embodiment 1, wherein in step (1), the probability $P_{SMK}$ is calculated by logistic regression analysis on the basis of data on the expression of all genes of 11 genes: TXNIP, EFNA1, ADM, SLC7A11, IGFBP3, WNT5A, HILPDA, EGLN3, CXCR4, AREG, and FBXO32.

[Embodiment 3] The in vitro method according to Embodiment 1 or 2, wherein in step (2), the probability $P_{COPD}$ is calculated on the basis of data on the expression of all genes of 4 genes:
EFNA1, TXNIP, DUSP6, and AREG,
with use of coefficients calculated in advance by logistic regression analysis using public data.

[Embodiment 4] The in vitro method according to any one of Embodiments 1 to 3, wherein the airway epithelial cells are mammalian airway epithelial cells.

[Embodiment 5] The in vitro method according to any one of Embodiments 1 to 4, wherein the airway epithelial cells are human airway epithelial cells, mouse airway epithelial cells, or rat airway epithelial cells.

[Embodiment 6] The in vitro method according to any one of Embodiments 1 to 5, wherein the airway epithelial cells are three-dimensional cultured cells.

[Embodiment 7] The in vitro method according to any one of Embodiments 1 to 6, wherein the airway epithelial cells are under air-liquid interface culture.

[Embodiment 8] The method according to any one of Embodiments 1 to 7, wherein when the PRF in the airway epithelial cells exposed to a solution containing an aerosol or particulate matter in the aerosol from a heated tobacco at a maximum concentration of 2000 puffs/L for 24 hours is in the range of 0.8 or less, it is determined that the risk of chronic obstructive pulmonary disease associated with the inhalation of the heated tobacco product is low or absent.

[Embodiment 9] The method according to any one of Embodiments 1 to 8, wherein the PRFs of two types of heated tobacco products are compared, and it is determined that the one with lower PRF has the risk of chronic obstructive pulmonary disease associated with inhalation lower than that of the other.

[Embodiment 10]
Use of

(1) data on the expression of 11 or more genes including at least the following 11 genes:
TXNIP, EFNA1, ADM, SLC7A11, IGFBP3, WNT5A, HILPDA, EGLN3, CXCR4, AREG, and FBXO32 from among the following 15 genes:
TXNIP, EFNA1, CYP1B1, ADM, AREG, DUSP6, SLC7A11, IGFBP3, WNT5A, CXCR4, PHLDA1, HILPDA, ZBED2, EGLN3, and FBXO32, and
(2) data on the expression of 4 or more genes including at least the following 4 genes:
EFNA1, TXNIP, DUSP6, and AREG
from among the following 15 genes:
TXNIP, EFNA1, CYP1B1, ADM, AREG, DUSP6, SLC7A11, IGFBP3, WNT5A, CXCR4, PHLDA1, HILPDA, ZBED2, EGLN3, and FBXO32,
for an in vitro method for evaluating the risk of chronic obstructive pulmonary disease associated with inhalation.

[Embodiment 11] A heated tobacco product having a structural feature of electrically heating directly or indirectly an aerosol source comprising reconstituted tobacco, wherein
when the in vitro method according to any one of Embodiments 1 to 6 is performed using a sample solution containing an aerosol or particulate matter in the aerosol from the heated tobacco, PRF is in the range of 0.8 or less in airway epithelial cells exposed to the solution containing an aerosol or the particulate matter in the aerosol from the heated tobacco product at a maximum concentration of 2000 puffs/L for 24 hours.

[Embodiment 12] The heated tobacco product according to Embodiment 11, wherein the reconstituted tobacco product is in the form of reconstituted tobacco granules having an average particle size of 0.2 mm or less, and the reconstituted tobacco granules are heated by a mixed vapor of propylene glycol and glycerin produced by heating all or part of a carrier comprising a porous substance or a fiber with an electric heater.

[Embodiment 13] The heated tobacco product according to Embodiment 11, wherein the reconstituted tobacco is a reconstituted tobacco sheet having an average width of 1 mm or more and an average length of 5 mm or less, and is wrapped in rolling paper to form a tobacco rod, and

the tobacco rod is in contact with the circumference of the rolling paper, and thus is heated by an electric heater.

[Embodiment 14] The heated tobacco product according to any one of Embodiments 11 to 13, wherein an aerosol produced contains acetaldehyde in an amount of 1.4 μg/rod or less.

[Embodiment 15] The heated tobacco product according to any one of Embodiments 11 to 13, wherein an aerosol produced contains acrolein in an amount of 1.2 μg/rod or less.

[Embodiment 16] The heated tobacco product according to any one of Embodiments 11 to 13, wherein an aerosol produced contains formaldehyde in an amount of 68.4 μg/rod or less.

ADVANTAGEOUS EFFECTS OF INVENTION

The in vitro method for evaluating the risk of chronic obstructive pulmonary disease associated with smoking or inhalation of the present invention enables statistic and quantitative evaluation and comparison of the risks of chronic obstructive pulmonary disease associated with smoking or inhalation. No method for statistically quantitatively evaluating the risk of chronic obstructive pulmonary disease associated with smoking or inhalation has been known in the past. The present invention has made it for the first time to statistically quantitatively evaluate and compare the risk. This is also of great scientific and industrial significance. The in vitro method for evaluating the risk of chronic obstructive pulmonary disease associated with smoking or inhalation of the present invention enables research and development of products with reduced risks (products that may have reduced health risks associated with the uses thereof, including smoking or inhalation).

BRIEF DESCRIPTION OF DRAWINGS

Fig. 1 shows an exemplary temperature changes due to the heating of a heated tobacco product.
Fig. 2 shows another exemplary temperature changes due to the heating of a heated tobacco product.
Fig. 3 shows the results of analyzing microarray data. (A) shows hierarchical clustering of characteristics of gene expression resulting from exposure to positive control reagents associated with biological responses that are suspected to be involved in COPD onset. (b) is a Venn diagram of an expression-variable gene resulting from exposure to whole smoke bubbling of a combustible cigarette.
Fig. 4 shows the calculation results of potential COPD risk values using the public database.
Fig. 5 shows the calculation results of potential COPD risk values in terms of mean $\pm$ standard deviation, the results obtained by 24 hours of exposure to the whole smoke bubbling of heated tobacco products (NGP1, NGP2) and standard reference cigarettes (3R4F, 1R6F).

DESCRIPTION OF EMBODIMENTS

1. In vitro method for evaluating the risk of chronic obstructive pulmonary disease associated with smoking or inhalation

The present invention relates to an in vitro method for evaluating the risk of chronic obstructive pulmonary disease associated with smoking or inhalation. The evaluation method comprises:

(1) calculating the following probability $P_{SMK}$ on the basis of data (specifically, microarray data or gene expression data obtained by a qPCR method) on the expression of 11 or more genes including at least the following 11 genes: TXNIP, EFNA1, ADM, SLC7A11, IGFBP3, WNT5A, HILPDA, EGLN3, CXCR4, AREG, and FBXO32 from among the following 15 genes in airway epithelial cells exposed to a sample solution containing an aerosol or particulate matter in the aerosol from a tobacco product:
TXNIP, EFNA1, CYP1B1, ADM, AREG, DUSP6, SLC7A11, IGFBP3, WNT5A, CXCR4, PHLDA1, HILPDA, ZBED2, EGLN3, and FBXO32,
with use of an intercept and coefficients calculated in advance by logistic regression analysis using public data;

[Chemical Formula 4]

$$logit(P_{SMK}) = ln\left(\frac{P_{SMK}}{1-P_{SMK}}\right) = C_{NS/SMK} + \beta_1 a_1 + \beta_2 a_2 + \cdots + \beta_x a_x$$

wherein $C_{NS|SMK}$ represents the intercept, $\beta_i$ represents the coefficient for each gene, $a_i$ represents gene expression data and represents a value after normalization of microarray data by GC-RMA or a quantitative value of gene expression obtained by the qPCR method, and x represents any integer of 11 to 15;

(2) calculating the following probability $P_{COPD}$ on the basis of data (specifically, microarray data or gene expression data obtained by the qPCR method) on the expression of 4 or more genes including at least the following 4 genes: EFNA1, TXNIP, DUSP6, and AREG

from among the following 15 genes in airway epithelial cells exposed to a sample solution containing an aerosol or particulate matter in the aerosol from a tobacco product:

TXNIP, EFNA1, CYP1B1, ADM, AREG, DUSP6, SLC7A11, IGFBP3, WNT5A, CXCR4, PHLDA1, HILPDA, ZBED2, EGLN3, and FBXO32,

with use of an intercept and coefficients calculated in advance by logistic regression analysis using public data;

[Chemical Formula 5]

$$logit(P_{COPD}) = ln\left(\frac{P_{COPD}}{1 - P_{COPD}}\right) = C_{SMK/COPD} + \gamma_1 b_1 + \gamma_2 b_2 + \cdots + \gamma_y b_y$$

wherein $C_{SMK|COPD}$ represents the intercept, $\gamma_i$ represents the coefficient for each gene, $b_i$ represents gene expression data and represents a value after normalization of microarray data by GC-RMA or a quantitative value of gene expression obtained by the qPCR method, and y represents any integer of 4 to 10; and

(3) calculating the risk of chronic obstructive pulmonary disease associated with smoking or inhalation of the smoke or the aerosol from the tobacco product, in terms of the following potential risk factor (PRF), from the probability $P_{SMK}$ and the probability $P_{COPD}$:

[Chemical Formula 6]

$$PRF = \frac{p_{SMK} \times p_{COPD}}{1 - (p_{SMK} \times p_{COPD})}$$

"Chronic Obstructive Pulmonary Disease (COPD)" is an inflammatory respiratory disease caused by long-term inhalation exposure to harmful substances such as air pollutants, dust, and microorganisms. COPD is thought to be closely related to smoking. The present invention relates to an in vitro statistical quantitative method for evaluating the risk of chronic obstructive pulmonary disease associated with smoking or inhalation.

The type of "tobacco product" is not particularly limited. The tobacco product includes combustible smoking articles such as cigarettes and also "heated tobacco products" which produces an aerosol by heating rather than burning. A heated tobacco product is a flavor inhaler that includes a tobacco rod portion. In a heated tobacco product, a porous or fibrous substance impregnated with alcohol, ethanol, glycerin, propylene glycol, or a mixed solution thereof is directly or indirectly heated with an electric heat source or a heat source based on a chemical reaction without burning the tobacco, and then a gas that is a mixture of the thus produced vapor and particulate matter is mixed into the air sucked through the flavor inhaler. Furthermore, as the gaseous mixture of vapor, particulate matter, and air passes through the tobacco, tobacco leaves, reconstituted tobacco, a reconstituted tobacco sheet, or reconstituted tobacco granules, a gas containing a smoking flavor component contained in tobacco, leaf tobacco, or reconstituted tobacco and volatile compounds such as nicotine and various natural flavors is mixed thereinto. With heated tobacco, a consumer inhales the gaseous mixture containing the volatile compound-containing gas, that is, the gaseous mixture of an aerosol (whole smoke) and a gas phase component. Usually, a consumer inhales the gaseous mixture by sucking at an end (mouth end or filter end, mouthpiece end) of the heated tobacco product.

A heated tobacco product includes three modes according to the aerosol production methods: a direct heating type flavor inhaler; an indirect heating type (air heating) flavor inhaler; and an indirect heating type (vapor heating) flavor inhaler. The direct heating type flavor inhaler is a flavor inhaler including a tobacco rod having the following features. The tobacco rod portion containing tobacco leaves, reconstituted tobacco, or a reconstituted tobacco sheet is directly heated mainly by electric heating to produce an aerosol containing leaf tobacco-derived nicotine and flavors. The indirect heating type (air heating) flavor inhaler is a flavor inhaler including a tobacco rod having the following features. The tobacco rod portion containing tobacco leaves, reconstituted tobacco, or a reconstituted tobacco sheet is indirectly heated by air heated by the use of a heat source such as electric heating to produce an aerosol containing leaf tobacco-derived nicotine and flavors. In the direct heating type flavor inhaler and the indirect heating type (air heating) flavor inhaler, volatile compounds are released from the tobacco leaves, the reconstituted tobacco, or the reconstituted tobacco

6

sheet and are mixed into the air sucked through the flavor inhaler, during inhalation. As the released compounds cool, they condense to form an aerosol, which is inhaled by the consumer. The indirect heating type (vapor heating) flavor inhaler is a flavor inhaler including a tobacco rod having the following features. An aerosol source (liquid) is heated by electric heating to produce an aerosol, resulting in the production of an aerosol containing leaf tobacco-derived nicotine and flavors.

"Aerosol from a tobacco product" refers to, for example, all components contained in the smoke of a cigarette, or, an aerosol produced from a flavor inhaler; specifically, an aerosol that is a gaseous mixture of gaseous substances, volatile substances, and particulate matter.

"Particulate matter in the aerosol from a tobacco product" refers to, for example, all substances except gaseous and volatile substances in the smoke of cigarettes or aerosols produced from flavor inhalers, and may also be referred to as total particulate matter (TPM). In the tobacco industry, the total particulate matter refers to a substance that is collected on/in a glass filter when the smoke of a cigarette or an aerosol produced from a flavor inhaler passes through the glass filter. In an embodiment, the particulate matter in a heated tobacco aerosol includes water vapor, nicotine, propylene glycol, glycerin, menthol, and others.

"Exposure to a sample solution containing particulate matter in the aerosol" includes a case of exposing cells to a sample solution in which total particulate matter is dissolved in an appropriate solvent or an appropriate liquid medium for cells, and also a case of exposing cells to a sample solution obtained by a whole smoke bubbling method in which the aerosol from a tobacco product, specifically a gaseous mixture of gaseous substances, volatile substances, and particulate matter is allowed to pass through an appropriate solvent or an appropriate liquid medium to dissolve the gaseous substances, the volatile substances, and the particulate matter therein.

As for a sample solution, the "solution" is not particularly limited as long as it is a solution in which an aerosol or particulate matter in the aerosol from a tobacco product can be dissolved. In an embodiment, an amphipathic solvent is used as the solution. Examples of the amphipathic solvent include, but are not limited to, dimethyl sulfoxide (DMSO), ethanol, methanol, isopropanol, and acetone.

The in vitro method for evaluating the risk of chronic obstructive pulmonary disease associated with smoking or inhalation utilizes airway epithelial cells exposed to a sample solution containing an aerosol or particulate matter in the aerosols produced from tobacco products. The concentration of an aerosol or particulate matter in the aerosol from a tobacco product in a sample solution, and the time for exposing the airway epithelial cells can be appropriately adjusted according to the type of the tobacco product and others. As used herein, the act of enjoying a tobacco product may be expressed by the term "smoking", which is used as a general term for the act of using, i.e., "smoking" or "inhaling", the tobacco product, including "inhalation".

"Airway epithelial cells" are cells derived from the epithelial tissue of the trachea-bronchi-bronchiole (sometimes collectively referred to as the "airway"), which is the transport route for expired air and intake air. Without limitation, the airway epithelial cells may be mammalian airway epithelial cells. Without limitation, the airway epithelial cells may be human airway epithelial cells, mouse airway epithelial cells, guinea pig airway epithelial cells, or rat airway epithelial cells.

In an embodiment, the airway epithelial cells are three-dimensional cultured cells. Three-dimensional cultured cells enable in vitro evaluation in an environment closer to the environment of organisms compared to two-dimensional cultured cells. For example, MucilAir™, available from Epithelix Sarl, is a human airway epithelial in vitro 3D reconstruction model reconstructed in a three-dimensional cell culture plate and can be used for the in vitro evaluation method.

In an embodiment, airway epithelial cells are under air-liquid interface culture. When the airway epithelial cells are under air-liquid interface culture, it is possible to induce the differentiation of the airway epithelial cells into ciliated cells, and to obtain an advantage that the state of the actual exposure can be reproduced in which the particulate matter is in direct contact with the airway epithelial cells.

The in vitro method for evaluating the risk of chronic obstructive pulmonary disease associated with smoking or inhalation comprises:

(1) calculating the following probability $P_{SMK}$, which is the probability of being determined as a smoker, on the basis of data (specifically, microarray data or gene expression data obtained by a qPCR method) on the expression of 11 or more genes including at least the following 11 genes:
TXNIP, EFNA1, ADM, SLC7A11, IGFBP3, WNT5A, HILPDA, EGLN3, CXCR4, AREG, and FBXO32 from among the following 15 genes in airway epithelial cells exposed to a sample solution containing an aerosol or particulate matter in the aerosol from a tobacco product:
TXNIP, EFNA1, CYP1B1, ADM, AREG, DUSP6, SLC7A11, IGFBP3, WNT5A, CXCR4, PHLDA1, HILPDA, ZBED2, EGLN3, and FBXO32,
with use of an intercept and coefficients calculated in advance by logistic regression analysis using public data;

[Chemical Formula 7]

$$logit(P_{SMK}) = ln\left(\frac{P_{SMK}}{1 - P_{SMK}}\right) = C_{NS/SMK} + \beta_1 a_1 + \beta_2 a_2 + \cdots + \beta_x a_x$$

wherein $C_{NS|SMK}$ represents the intercept, $\beta_i$ represents the coefficient for each gene, $a_i$ represents gene expression data, and represents a value after normalization of microarray data by GC-RMA or a quantitative value of gene expression obtained by the qPCR method, and x represents any integer of 11 to 15.

The following 15 genes are genes found to be associated with smoking in the present invention. TXNIP, EFNA1, CYP1B1, ADM, AREG, DUSP6, SLC7A11, IGFBP3, WNT5A, CXCR4, PHLDA1, HILPDA, ZBED2, EGLN3, and FBXO32

Of these, the following 11 genes are genes found to be particularly highly associated with smoking by the backward elimination.

TXNIP, EFNA1, ADM, SLC7A11, IGFBP3, WNT5A, HILPDA, EGLN3, CXCR4, AREG, and FBXO32

Examples of the microarray data of the public database that can be used include, but are not limited to, the microarray data registered in EMBL-EBI: E-MTAB-1690, E-GEOD-20257, and E-GEOD-8545.

Regarding logistic regression analysis using public data, for example, the groups of healthy subjects (HLT) and smokers (SMK) are used as objective variables from the attribute information given to the microarray data registered in the public database; normalized gene expression data $a_i$ obtained by GC-RMA performed for genes including 11 genes, TXNIP, EFNA1, ADM, SLC7A11, IGFBP3, WNT5A, HILPDA, EGLN3, CXCR4, AREG, and FBXO32 are extrapolated as explanatory variables; and then, without limitation, logistic regression analysis is performed using, for example, the statistical analysis free software "R," so that the intercept $C_{NS|SMK}$ and the coefficients $\beta_i$ can be obtained from the obtained regression equation.

In an embodiment, "11 or more genes including at least the following 11 genes: TXNIP, EFNA1, ADM, SLC7A11, IGFBP3, WNT5A, HILPDA, EGLN3, CXCR4, AREG, and FBXO32" are 11, 12, 13, 14, or 15 genes. Preferably, these genes are 11 genes.

The in vitro evaluation method also comprises (2) calculating the following probability $P_{COPD}$, which is the probability of discriminating COPD patients from the other smokers, on the basis of data (specifically, microarray data or gene expression data obtained by the qPCR method) on the expression of 4 or more genes including at least the following 4 genes: EFNA1, TXNIP, DUSP6, and AREG

from among the following 15 genes in airway epithelial cells exposed to a sample solution containing an aerosol or particulate matter in the aerosol from a tobacco product:

TXNIP, EFNA1, CYP1B1, ADM, AREG, DUSP6, SLC7A11, IGFBP3, WNT5A, CXCR4, PHLDA1, HILPDA, ZBED2, EGLN3, and FBXO32,

with use of an intercept and coefficients calculated in advance by logistic regression analysis using public data;

[Chemical Formula 8]

$$logit(P_{COPD}) = ln\left(\frac{P_{COPD}}{1 - P_{COPD}}\right) = C_{SMK/COPD} + \gamma_1 b_1 + \gamma_2 b_2 + \cdots + \gamma_y b_y$$

wherein $C_{SMK|COPD}$ represents the intercept, $\gamma_i$ represents the coefficient for each gene, $b_i$ represents gene expression data and represents a value after normalization of microarray data by GC-RMA or a quantitative value of gene expression obtained by the qPCR method, and y represents any integer of 4 to 10.

The 4 genes, EFNA1, TXNIP, DUSP6, and AREG are genes highly associated with COPD as found by the backward elimination. DUSP6 is not included in the 11 genes that are particularly highly associated with smoking, but is included in the 15 genes found to be associated with smoking.

Examples of the microarray data of the public database that can be used include, but are not limited to, the microarray data registered in EMBL-EBI: E-MTAB-1690, E-GEOD-20257, and E-GEOD-8545.

Regarding the logistic regression equation using public data, for example, the groups of smokers (SMK) and COPD patients (COPD) are used as objective variables from the attribute information given to the microarray data registered in the public database; normalized gene expression data $b_i$ obtained by GC-RMA performed for genes including 4 genes, EFNA1, TXNIP, DUSP6, and AREG are extrapolated as explanatory variables; and then, without limitation, logistic regression analysis is performed using, for example, the statistical analysis free software "R," so that the intercept $C_{SMK|COPD}$ and the coefficients $\gamma_i$ can be obtained from the obtained regression equation.

In an embodiment, the number of "4 or more genes including EFNA1, TXNIP, DUSP6, and AREG" are 4, 5, 6, 7, 8, 9, or 10 genes. The number is preferably 4 genes.

Then, the in vitro evaluation method comprises (3) calculating the risk of chronic obstructive pulmonary disease associated with smoking or inhalation, in terms of the following potential risk factor (PRF), from the probability $P_{SMK}$ and the probability $P_{COPD}$ on the basis of the results of (1) and (2):

[Chemical Formula 9]

$$PRF = \frac{p_{SMK} \times p_{COPD}}{1 - (p_{SMK} \times p_{COPD})}$$

In step (1) in an embodiment, the probability $P_{SMK}$ is calculated on the basis of microarray data on the expression of all 11 genes:
TXNIP, EFNA1, ADM, SLC7A11, IGFBP3, WNT5A, HILPDA, EGLN3, CXCR4, AREG, and FBXO32,
with use of intercepts and coefficients calculated in advance by logistic regression analysis using the public data.

In step (2) in an embodiment, the probability $P_{COPD}$ is calculated on the basis of microarray data on the expression of all 4 genes:
EFNA1, TXNIP, DUSP6, and AREG,
with use of intercepts and coefficients calculated in advance by logistic regression analysis using the public data.

In the Examples herein, upon 24 hours of exposure using NGPs (NEXT GENERATION PRODUCTS) (NGP1 and NGP2) at a maximum concentration of 2000 puffs/L, the PRF is 0.07 for non-smokers, 0.46 for smokers, and 0.83 for COPD patients, thus confirming an increase in PRF as compared with healthy non-smokers. NGP1 and NGP2 used in the Examples correspond to the "heated tobacco" according to "third embodiment" and the "heated tobacco" according to "second embodiment," respectively, as described later in the sections of "Embodiments of heated tobacco" herein. Details of NGP2 are also described in Japanese Patent Application No. 2018-213498 (unpublished).

In an embodiment of the in vitro evaluation method, when the PRF in the airway epithelial cells exposed to a solution containing an aerosol or particulate matter in the aerosol from a heated tobacco at a maximum concentration of 2000 puffs/L for 24 hours is in the range of 0.8 or less (lower than the PRFs for the COPD patients in the Examples), it is determined that the risk of chronic obstructive pulmonary disease associated with inhalation of the heated tobacco is low or absent. Without limitation, PRF can be calculated in the same manner when the same experiment is performed under the conditions of 24 hours of exposure at the minimum concentration of 200 puffs/L.

In an embodiment, when the PRF in the airway epithelial cells exposed to a solution containing an aerosol or particulate matter in the aerosol from a heated tobacco product at a maximum concentration of 2000 puffs/L for 24 hours is in the range of 0.4 or less (lower than the PRFs for smokers in the Examples), it is determined that the risk of chronic obstructive pulmonary disease associated with inhalation of the heated tobacco product is low or absent.

In an embodiment of the in vitro evaluation method, the PRFs of two types of heated tobacco products are compared, and it is determined that the one with lower PRF has the risk of chronic obstructive pulmonary disease associated with inhalation lower than that of the other.

The present invention also relates to the use of

(1) microarray data on the expression of 11 or more genes including at least the following 11 genes:
TXNIP, EFNA1, ADM, SLC7A11, IGFBP3, WNT5A, HILPDA, EGLN3, CXCR4, AREG, and FBXO32 from among the following 15 genes:
TXNIP, EFNA1, CYP1B1, ADM, AREG, DUSP6, SLC7A11, IGFBP3, WNT5A, CXCR4, PHLDA1, HILPDA, ZBED2, EGLN3, and FBXO32, and
(2) microarray data on the expression of 4 or more genes including at least the following 4 genes:
EFNA1, TXNIP, DUSP6, and AREG
from among the following 15 genes:
TXNIP, EFNA1, CYP1B1, ADM, AREG, DUSP6, SLC7A11, IGFBP3, WNT5A, CXCR4, PHLDA1, HILPDA, ZBED2, EGLN3, and FBXX32
for an in vitro method for evaluating the risk of chronic obstructive pulmonary disease associated with smoking or inhalation.

2. Heated tobacco product

The present invention relates to a heated tobacco product. The heated tobacco product has a structural feature of electrically heating directly or indirectly an aerosol source comprising reconstituted tobacco.

When the in vitro evaluation method of the present invention is performed using a sample solution containing an aerosol or particulate matter in the aerosol from the heated tobacco product, PRF is in the range of 0.8 or less, or PRF is in the range of 0.4 or less, in airway epithelial cells exposed to the solution containing an aerosol or the particulate matter in the aerosol from the heated tobacco product at a maximum concentration of 2000 puffs/L for 24 hours. PRF has been revealed for the first time by the in vitro method for evaluating the risk of chronic obstructive pulmonary disease associated with smoking or inhalation of the present invention. A heated tobacco product with such a low PRF was first conceived by the in vitro method for evaluating the risk of chronic obstructive pulmonary disease associated with smoking or inhalation of the present invention.

The embodiments of "heated tobacco product" are as described in the section" 1. In vitro method for evaluating the risk of chronic obstructive pulmonary disease associated with smoking or inhalation." The heated tobacco product has a structural feature of electrically heating directly or indirectly an aerosol source comprising reconstituted tobacco.

In an embodiment, the reconstituted tobacco of a heated tobacco product is in the form of reconstituted tobacco granules having an average particle size of 0.2 mm or more and 1.2 mm or less. In an embodiment, the reconstituted tobacco granules are heated by a vapor mixture of propylene glycol and glycerin produced by heating all or part of a carrier comprising a porous material or a fiber with an electric heater. In an embodiment, the reconstituted tobacco of a heated tobacco product is in the form of reconstituted tobacco granules having an average particle size of 0.2 mm or more and an average particle size of 1.2 mm or less. The reconstituted tobacco granules are heated by a vapor mixture of propylene glycol and glycerin produced by heating all or part of a carrier comprising a porous substance or a fiber with an electric heater.

In an embodiment, the reconstituted tobacco is a reconstituted tobacco sheet having an average width of 1 mm or more and an average length of 5 mm or less. In an embodiment, the reconstituted tobacco sheet is wrapped in rolling paper to form a tobacco rod. The tobacco rod is in contact with the circumference of the rolling paper, and thus is heated by an electric heater. In an embodiment, the reconstituted tobacco is a reconstituted tobacco sheet having an average width of 1 mm or more and an average length of 5 mm or less, and wrapped in rolling paper to form a tobacco rod. The tobacco rod is in contact with the circumference of the rolling paper, and thus is heated by an electric heater.

Embodiments of heated tobacco product

Hereinafter, embodiments of a heated tobacco product will be described without limitation.

The heated tobacco product is an article that generates an aerosol-gas mixture while being inserted into an aspirator equipped with an electric heater, the aerosol-gas mixture being subjected to inhalation through the oral cavity, respiratory tract, and nasal cavity of consumers. The shape is rod-shaped, and the total length may be 20 mm or more and 100 mm or less, or 50 mm or more and 85 mm or less.

In the first embodiment, the shape is rod-shaped, and the total length may be 20 mm or more, 30 mm or more, 40 mm or more, 50 mm or more, 60 mm or more, 70 mm or more, 80 mm or more, 83 mm, 50 mm or less, 60 mm or less, 70 mm or less, 80 mm or less, 90 mm or less, or 100 mm or less.

In the second embodiment, the shape is rod-shaped, and the total length may be 20 mm or more, 30 mm or more, 40 mm or more, 50 mm or more, 60 mm or more, 70 mm or more, 80 mm or more, 55 mm, 50 mm or less, 60 mm or less, 70 mm or less, 80 mm or less, 90 mm or less, or 100 mm or less. The outer circumference may be 15 mm or more, 20 mm or more, 25 mm or less, 30 mm or less, or 22 mm

In the third embodiment, the heated tobacco is a polypropylene plastic capsule containing reconstituted tobacco granules. The shape of the plastic capsule is substantially cylindrical, and the total length may be 10 mm or more, 20 mm or more, 30 mm or more, 40 mm or more, 50 mm or more, 60 mm or more, 70 mm or more, 40 mm or less, 50 mm or less, 60 mm or less, 70 mm or less, 80 mm or less, or 90 mm or less.

In both the first and second embodiments, the heated tobacco product comprises a tobacco rod portion, a cavity portion, a paper tube, and a filter portion, and the filter portion comprises a fiber section or a cavity section or a hollow paper tube section.

The heated tobacco product according to the first embodiment will be described.

Tobacco rod portion: The tobacco rod portion is rod-shaped, and the length thereof is 30 mm or more and 50 mm or less, preferably 42 mm in the long axis direction of the heated tobacco product. The tobacco rod portion is formed such that small pieces of a reconstituted tobacco sheet are wrapped in rolling paper.

Reconstituted tobacco: The small pieces obtained by cutting the reconstituted tobacco sheet may have a width of 1 mm or more and a length of 15 mm or less. The length may preferably be 10 mm or less, more preferably 5 mm or less. The reconstituted tobacco sheet may contain nicotine as a flavor component in an amount of 0.80% or more and 0.90% or less. The reconstituted tobacco sheet may contain menthol as a flavor component. In that case, menthol may be contained in an amount of 0.3 mg or more and 5.3 mg or less per tobacco rod portion. The reconstituted tobacco sheet may contain alcohol as a moisturizer, and may contain propylene glycol in an amount of 0.04% or more and 0.58% or less, and glycerin in an amount of 12.4% or more and 14.3% or less. The reconstituted tobacco sheet may contain sugars, monosaccharides, and disaccharides as a moisturizer. The reconstituted tobacco sheet may contain monosaccharides and disaccharides in an total amount of 3.7% or less, and may contain fructose in an amount of 2.1% or more and 2.7% or less, glucose in an amount of 1.3% or more and 1.8% or less, and sucrose in an amount of 0.2% or more and 0.4% or less. The reconstituted tobacco sheet may contain an organic acid as a pH adjuster, and may contain citric acid at 4.1 mg/g or more and 4.6 mg/g or less, malic acid at 30.9 mg/g or more and 34.4 mg/g or less, 30.9 mg/g or more and 34.4 mg/g or less, succinic acid at 0.2 mg/g or more and 0.3 mg/g or less, and formic acid at 0.1 mg/g or more and 0.2 mg/g or less.

Rolling paper: The rolling paper may have an air permeability of 64.0 (Corresta Unit) or more and 67.0 (Corresta Unit) or less, and may have a basis weight of 26.2 (g/m$^2$) or more and 26.4 (g/m$^2$) or less. The calcium carbonate content thereof may be 25.2% or more and 25.5% or less, and the combustion improver content thereof may be 0.7%.

Filter portion: The filter portion is rod-shaped, and the length thereof is preferably 5 mm or more and 18 mm or less in the long axis direction of the heated tobacco product. The filter portion comprises an acetate fiber filter and a hollow paper tube. The length of the acetate fiber filter may be 5 mm or more and 18 mm or less in the long axis direction, and is desirably 8 mm The acetate fiber constituting the acetate fiber filter may have an arbitrary cross-sectional shape, and may have a round cross-sectional shape, a Y-shaped cross-sectional shape, or an X-shaped cross-sectional shape. The length of the hollow paper tube may be 5 mm or more and 18 mm or less in the long axis direction, and is desirably 8 mm

Cavity portion: The cavity portion is defined by: the inner surface of the chip paper that extends from the end of the cavity portion on the filter portion side to the filter portion by 5 mm or more and 16 mm or less and extends from the end on the tobacco rod portion side to the tobacco rod portion by 5 mm or less; the end of the filter portion; and the ends of the cut reconstituted tobacco sheet that constitutes the tobacco rod. The cavity portion is rod-shaped, and the length thereof may be 20 mm or more and 30 mm or less in the long axis direction of the heated tobacco, and is desirably 25 mm

Chip paper: The chip paper constitutes a cavity portion and may be provided with a plurality of, i.e., two or more openings aligned in the peripheral direction. The row of the openings may be a single row, or a plurality of, i.e., two or more rows may be provided. The chip paper may be internally lined with paper tubes obtained by spirally molding tape-shaped pieces of paper and cutting them in such a manner that each piece has a fixed length.

The heated tobacco product according to the second embodiment will be described.

Tobacco rod portion: The tobacco rod portion is rod-shaped, and the length thereof is 15 mm or more and 25 mm or less, preferably 22 mm in the long axis direction of the heated tobacco product. The tobacco rod portion is formed such that small pieces of a reconstituted tobacco sheet are wrapped with rolling paper. The small pieces obtained by cutting the reconstituted tobacco sheet may have a width of 1 mm or more and a length of 15 mm or less, preferably a length of 10 mm or less, and more preferably 5 mm or less.

Reconstituted tobacco: Small pieces of the reconstituted tobacco sheet may be contained in the tobacco rod portion in an amount of 200 mg or more and 300 mg, 220 mg or more and 280 mg or less, 240 mg or more and 260 mg or less, 250 mg or more and 260 mg, 255 mg, or 260 mg.

Rolling paper: The rolling paper may have an air permeability of 64.0 (Corresta Unit) or more and 67.0 (Corresta Unit) or less, and may have a basis weight of 26.2 (g/m$^2$) or more and 26.4 (g/m$^2$) or less. The calcium carbonate content hereof may be 25.2% or more and 25.5% or less, and the combustion improver content thereof may be 0.7%.

Filter portion: The filter portion is rod-shaped, and the length thereof is 5 mm, 20 mm, or 30 mm or more and 40 mm or less, preferably 35 mm in the long axis direction of the heated tobacco product. The filter portion comprises an acetate fiber filter, a hollow paper tube, and a hollow acetate tube.

The length of the acetate fiber filter may be 5 mm or more and 35 mm or less in the long axis direction, and is desirably 7 mm The acetate fiber constituting the acetate fiber filter may have an arbitrary cross-sectional shape, and may have a round cross-sectional shape, a Y-shaped cross-sectional shape, or an X-shaped cross-sectional shape.

The length of the hollow paper tube may be 5 mm or more and 35 mm or less in the long axis direction, and is desirably 20 mm The hollow paper tube may be provided with a plurality of, i.e., two or more openings, desirably 15 openings, which are aligned in the peripheral direction, as outside air intake holes. The row of openings for the outside air intake holes may be a single row, or a plurality of, two or more rows may be provided.

The hollow acetate tube is an acetate tube made of acetate fiber and having a wall thickness of 1.2 mm, and the length of the hollow acetate tube may be 5 mm or more and 35 mm or less in the long axis direction, and is desirably 8 mm

Examples of the heated tobacco product according to the third embodiment include the non-combustible flavor inhaler described in WO2016/075748 and WO2016/075749. The non-combustible flavor inhaler includes a power supply unit, the first cartridge including an aerosol source and an atomizer portion, and the second cartridge. The second cartridge imparts flavor to an aerosol by passing the aerosol atomized in the atomizer portion therethrough. It should be noted here that flavor can be imparted to the aerosol without heating the flavor source. It should also be noted that substantially no aerosol is produced from the flavor source. The atomizer portion atomizes the aerosol source without burning. The second cartridge accommodates the flavor source that imparts flavor to the aerosol. The flavor source is composed of granules of reconstituted tobacco that imparts flavor to the aerosol produced by the non-combustible flavor inhaler. The size of the reconstituted tobacco granules is preferably 0.2 mm or more and 1.2 mm or less. Furthermore, the size of the reconstituted tobacco granules is preferably 0.2 mm or more and 0.7 mm or less. As the size of the reconstituted tobacco granules constituting the flavor source is smaller, the specific surface area is larger, and the smoking flavor components are thus more likely to volatilize from the granules constituting the flavor source.

Embodiments of heating

Hereinafter, embodiments of heating in a heated tobacco product will be described without limitation.

The heated tobacco product according to the first embodiment is heated by a single film-shaped heater having a set of two heating wires and coated with a resin to generate an aerosol-gas mixture. The heated tobacco product is cooled by outside air that is introduced into an aggregate of small pieces of reconstituted tobacco sheet from the end opposite to the mouthpiece end of the tobacco rod portion due to heating from the circumference by a heater and inhalation operation. Therefore, the temperature distribution of the aggregate of small pieces of the reconstituted tobacco sheet when the heated tobacco product generates an aerosol-gas mixture is not uniform. Specifically, thermocouples inserted at the following three positions: the position 1 mm away from the end on the side opposite to the mouthpiece (Top), the central position (Middle), and the position 1 mm away from the end on the mouthpiece side (End) exhibit temperature changes different from each other, and thermocouples even at the same position exhibit different modes of temperature changes between the peripheral portion (Peripheral) and the central portion (Center). Further, the positions other than End is cooled by the outside air introduced into the tobacco rod portion due to the inhalation operation, and the temperature drops in a short time at these positions.

At Peripheral-Top, the temperature change of a heated tobacco product is roughly divided into five phases. The first phase ranges from the start of heating to 40 seconds after the start of heating. The temperature rises to 75°C within 40 seconds. The second phase ranges from 40 seconds to 135 seconds. The temperature rises from 75°C to 150°C and is then maintained. The third phase ranges from 135 seconds to 150 seconds. The temperature rises from 150°C to 200°C. The fourth phase ranges from 150 seconds to 220 seconds. The temperature rises from 200°C to 230°C. The final phase is 220 seconds or later. The temperature drops from 230°C to room temperature.

At Peripheral-Middle, the temperature change of a heated tobacco product is roughly divided into five phases. The first phase ranges from the start of heating to 25 seconds after the start of heating. The temperature rises to 150°C. The second phase ranges from 25 seconds to 135 seconds. The temperature rises from 150°C to 200°C. The third phase ranges from 135 seconds to 150 seconds. The temperature rises from 200°C to 230°C. The fourth phase ranges from 150 seconds to 220 seconds. The temperature rises from 230°C to 240°C. The final phase is 220 seconds or later. The temperature drops from 240°C to room temperature.

At Peripheral-End, the temperature change of a heated tobacco product is roughly divided into four phases. The first phase ranges from the start of heating to 25 seconds after the start of heating. The temperature rises to 200°C within 25 seconds. The second phase ranges from 25 seconds to 150 seconds. The temperature rises from 200°C to 240°C and is then maintained. The third phase ranges from 150 seconds to 220 seconds. The temperature drops from 240°C to 200°C. The final phase is 220 seconds or later. The temperature drops from 200°C to room temperature.

At Center-Top, the temperature change of a heated tobacco product is roughly divided into five phases. The first phase ranges from the start of heating to 40 seconds after the start of heating. The temperature rises to 50°C. The second phase ranges from 40 seconds to 135 seconds. The temperature rises from 50°C to 125°C. The third phase ranges from 135 seconds to 150 seconds. The temperature rises from 150°C to 180°C. The fourth phase ranges from 150 seconds to 220 seconds. The temperature rises from 200°C to 210°C. The final phase is 220 seconds or later. The temperature drops from 210°C to room temperature.

At Center-Middle, the temperature change of a heated tobacco product is roughly divided into five phases. The first phase ranges from the start of heating to 25 seconds after the start of heating. The temperature rises to 100°C. The second phase ranges from 25 seconds to 135 seconds. The temperature rises from 100°C to 180°C. The third phase ranges from 135 seconds to 150 seconds. The temperature rises from 180°C to 200°C. The fourth phase ranges from 150 seconds to 220 seconds. The temperature rises from 200°C to 240°C. The final phase is 220 seconds or later. The temperature drops from 240°C to room temperature.

At Center-End, the temperature change of a heated tobacco product is roughly divided into four phases. The first phase ranges from the start of heating to 25 seconds after the start of heating. The temperature rises to 100°C. The second phase ranges from 25 seconds to 45 seconds. The temperature rises from 100°C to 150°C. The third phase ranges from 45 seconds to 135 seconds. The temperature rises from 150°C to 230°C. The fourth phase ranges from 135 seconds to 220 seconds. The temperature drops from 230°C to 200°C. The final phase is 220 seconds or later. The temperature drops from 200°C to room temperature. The temperature change described above is continuous through the transition of the phases.

The heating of a heated tobacco according to the second embodiment can be performed in the same manner as that of the first heated tobacco.

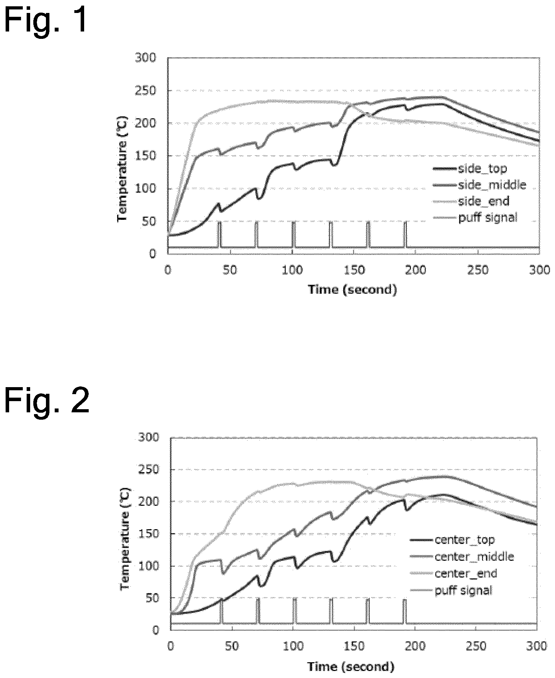

In an example, a heater that causes the above-mentioned temperature changes in the heated tobacco product of the first and second embodiments may exhibit the modes of temperature changes as shown in Figs. 1 and 2.

The temperature change of the heater at the first location may be roughly divided into four phases. The first phase ranges from the start of heating to 25 seconds after the start of heating. The temperature of the heater rises to 200°C within 25 seconds after the start of inhalation. The second phase ranges from 25 seconds to 150 seconds. The temperature of the heater rises to 230°C from 30 seconds to 45 seconds, and is thereafter maintained at about 230°C until the passage of 150 seconds. The third phase ranges from 150 seconds to 220 seconds. The temperature of the heater drops to 220°C at 150 seconds or later and is maintained at 220°C until the passage of 220 seconds. The final phase is 220 seconds or later. The temperature of the heater drops from 220°C to normal temperature.

The temperature change of the heater at the second location may be roughly divided into four phases. The first phase is from the start of inhalation to 75 seconds after the start of inhalation. The temperature of the heater rises to 100°C within 75 seconds after the start of inhalation. The second phase ranges from 75 seconds to 135 seconds. The temperature of the heater rises to 140°C from 75 seconds to 80 seconds, and thereafter rises to 150°C until the passage of 135 seconds. The third phase ranges from 135 seconds to 220 seconds. The temperature of the heater rises to 230°C from 135 seconds to 150 seconds and is maintained at 230°C from 150 seconds to 220 seconds. The final phase is 220 seconds or later. The temperature of the heater drops from 230°C to normal temperature. The temperature changes at the first and second locations are continuous through the transition of the phases.

The heating of the heated tobacco product according to the third embodiment is performed as follows, for example. The atomizer portion includes a heating wire, an electromagnetic induction heating mechanism, or an ultrasonic oscillator, and is supplied with current from the power supply unit during the inhalation operation by users to generate heat or operate to atomize the aerosol source included in the first cartridge. Flavor is imparted to the aerosol atomized in the atomizer portion by the passage of the aerosol. When the atomized aerosol passes through the second cartridge, the temperature of the atmosphere in the second cartridge is normal temperature or higher, or 26°C or higher and 40°C or lower, preferably 30°C or lower. It should be noted here that the flavor source can impart flavor to the aerosol without being heated.

Aerosol-gas mixture component of heated tobacco product

Hereinafter, embodiments of the aerosol-gas mixture component of a heated tobacco product will be described without limitation.

In the aerosol-gas mixture generated by 6 or 8 smoking operations under smoking conditions (CIR conditions) specified by Health Canada, provided that the openings provided in the filter portion of the heated tobacco product are not blocked, the amount of a carbonyl compound per tobacco rod may be those in the following embodiments.

In the first embodiment, the amount of formaldehyde is preferably 100.00 ($\mu$g/rod) or less. Desirably the amount is preferably 70.00 ($\mu$g/rod) or less. More desirably, the amount is preferably 50.00 ($\mu$g/rod) or more and 70.00 ($\mu$g/rod) or less.

Most desirably, the amount may be 57.60 ($\mu$g/rod) or more and 68.40 ($\mu$g/rod) or less.

In the second embodiment, the amount of formaldehyde is preferably 40.00 ($\mu$g/rod) or less. More desirably, the amount is 37.00 ($\mu$g/rod) or less.

In the first embodiment, the amount of acetaldehyde is preferably 3.000 ($\mu$g/rod) or less. Desirably, the amount is preferably 2.000 ($\mu$g/rod) or less. More desirably, the amount is preferably 0.750 ($\mu$g/rod) or more and 2.000 ($\mu$g/rod) or less. Most desirably, the amount may be 0.800 ($\mu$g/rod) or more and 1.400 ($\mu$g/rod) or less.

In the second embodiment, the amount of acetaldehyde is preferably 1.000 ($\mu$g/rod) or less. More desirably, the amount is preferably 0.940 ($\mu$g/rod) or less.

In the first embodiment, the amount of acrolein is preferably 3.000 ($\mu$g/rod) or less. Desirably, the amount is preferably 2.000 ($\mu$g/rod) or less. More desirably, the amount is preferably 0.750 ($\mu$g/rod) or more and 1.500 ($\mu$g/rod) or less. Most preferably, the amount may be 0.800 ($\mu$g/rod) or more and 1.200 ($\mu$g/rod) or less.

In the second embodiment, the amount of acrolein is preferably 0.300 ($\mu$g/rod) or less. More desirably, the amount is preferably 0.265 ($\mu$g/rod) or less.

In the third embodiment, the amounts of formaldehyde, acrolein, and acetaldehyde are below the detection limit, and are 0.000 ($\mu$g/rod) as determined by the current analytical techniques.

In the aerosol-gas mixture generated by 8 smoking operations under smoking conditions (CIR conditions) specified by Health Canada, provided that the openings provided in the filter portion of the heated tobacco product are not blocked, the content of TSNAs per tobacco rod may be 10% or less based on that of 3R4F cigarette smoked under the smoking conditions (CIR conditions) specified by Health Canada. The content of formaldehyde may be 0% or more, or 0.80% or more, and 1.30% or less. The content of acetaldehyde may be 0% or more, or 3.30% or more, and 3.80% or less. The content of acrolein may be 0% or more, or 0.40% or more, and 0.70% or less.

In one embodiment, the produced heated tobacco aerosol contains acetaldehyde in an amount of 1.4 $\mu$g/rod

or less.

In one embodiment, the produced heated tobacco aerosol contains acrolein in an amount of 1.2 µg/rod or less.

In one embodiment, the produced heated tobacco aerosol contains formaldehyde in an amount of 68.4 µg/rod or less.

In the aerosol-gas mixture generated by 6 or 8 smoking operations under smoking conditions (CIR conditions) specified by Health Canada, provided that the openings provided in the filter portion of the heated tobacco product are not blocked, the amounts of nicotine, glycerin, and propylene glycol per tobacco rod may be those in the following embodiments.

In the first embodiment, the amount of nicotine may be 0.240 mg/rod or more and 0.510 mg/rod or less, that of glycerin may be 0.700 mg/rod or more and 2.900 mg/rod or less, and that of propylene glycol may be 0.020 mg/rod or more and 0.270 mg/rod or less.

In the second embodiment, the amount of nicotine may be 0.400 mg/rod or more and 0.500 mg/rod or less, that of glycerin may be 2.000 mg/rod or more and 2.500 mg/rod or less, and that of propylene glycol may be 0.400 mg/rod or more and 0.500 mg/rod or less.

In the third embodiment, in the aerosol-gas mixture generated by 50 smoking operations in accordance with the CRM method (CORRESTA RECOMMENDED METHOD No. 81) conditions, the amount of nicotine may be 1.000 mg/rod or more and 1.500 mg/rod or less, that of glycerin may be 20.00 mg/rod or more and 30.00 mg/rod or less, and that of propylene glycol may be 40.00 mg/rod or more and 60.00 mg/rod or less.

The present invention is an in vitro method for evaluating the risk of chronic obstructive pulmonary disease associated with smoking or inhalation, and is intended to provide a method for statistically quantitatively evaluating risk of chronic obstructive pulmonary disease associated with smoking or inhalation, which is conventionally-unknown. By the use of the method for statistically quantitatively evaluating the risk of chronic obstructive pulmonary disease associated with smoking or inhalation of the present invention, a risk-reducing product with reduced risks as described above (a heated tobacco product that may have reduced health risks associated with smoking or inhalation) is provided.

EXAMPLES

Hereinafter, the present invention will be described in detail based on Examples, but the present invention is not limited to these Examples. Those skilled in the art can easily make modifications and changes to the present invention based on the description herein, and those are included in the technical scope of the present invention.

Example 1 Cell culture

Human airway epithelial cells MucilAir™ were purchased from Epithelix Sarl. The cells cultured for 10 days under an environment of 37°C and 5% $CO_2$ were used for the test. Medium exchange was performed every 2 to 3 days during culture.

Example 2 Microarray test

It was hypothesized that COPD onset is caused by oxidative stress derived from active oxygen, an inflammatory response associated with long-term oxidative stress, and DNA damage. An exposure experiment was conducted using substances that induce these biological responses as positive control reagents.

Specifically, first, in a single-exposure test, a substance from NaClO, tBHQ, TNFα, IL-1β, cisplatin, and bleomycin was added as a positive control reagent to MucilAir™ at an arbitrary concentration. NaClO and tBHQ induce oxidative stress, TNFα and IL-1β induce inflammatory response, and cisplatin and bleomycin induce DNA damage, as responses. mRNA was extracted 4 hours after the addition of the substance. RNeasy from Qiagen N.V. was used for mRNA extraction. After mRNA extraction, microarray data was obtained using Human Genome U133 Plus 2.0 arrays (Affymetrix, Inc.) (data 1: positive control reagent exposure data).

A standard test cigarette 3R4F was used as a reference cigarette, and mainstream smoke generated by a method in accordance with ISO (International Organization for Standardization) (ISO: 3308) was allowed to pass through a medium and collected (whole smoke bubbling solution). Whole smoke bubbling solutions having adjusted concentrations of 0.5 cigarette/L, 1.0 cigarette /L, and 2.0 cigarettes /L were prepared, wherein the cigarette means 3R4F. The solution was added to cells, followed by mRNA extraction and microarray data acquisition, by the same method as above (data 2: 3R4F reference cigarette exposure data).

Next, in a frequent exposure test, mainstream smoke generated from 3R4F and mainstream smoke generated from heated tobacco product according to the third embodiment by the ISO method and the CRM method (CORRESTA RECOMMENDED METHOD No. 81) were collected and the obtained whole smoke bubbling solutions were diluted, so that the multiple concentrations of test solutions were adjusted (3R4F: 25-200 puffs/L, 1R6F: 50-200 puffs/L, NGP1:

250-2000 puffs/L, NGP2: 200-2000 puffs/L).

Regarding heated tobacco product, the amount of nicotine to be used for exposure of cells was adjusted to be almost the same as or higher than that of 3R4F and that of 1R6F by collecting the smoke in a manner such that the frequency of collection was 10 times the frequency of collecting the smoke from the control samples of 3R4F and 1R6F. That is, each sample was prepared in which the total particulate matter to be used for exposure of cells was 10 times more than that of the control samples of 3R4F and 1R6F.

These test solutions were added to MucilAir™ and exposure was performed for 24 hours. After the exposure, mRNA was extracted and microarray data was obtained (data 3: 3R4F reference cigarette & NGP1 exposure data, data 4: 1R6F reference cigarette & NGP2 exposure data).

Example 3 Microarray data analysis

Data 1: positive control reagent exposure data, data 2: 3R4F reference cigarette data, data 3: 3R4F reference cigarette & NGP1 exposure data, and data 4: 1R6F reference cigarette & NGP2 exposure data, which were obtained in Example 2, and the CEL files of microarray data (E-MTAB-1690, E-GEOD-20257, and E-GEOD-8545) in the public database were normalized for each test using GeneSpring ver. 14.9 (Agilent Technologies, Inc.). As a normalization algorithm, GC-Robust Multiarray Average (GC-RMA) was used, and baseline correction was performed using the average value of all samples. The normalized data were subjected to gene name annotation using the statistical analysis free software "R."

Example 4 Data analysis

In this Example, the microarray data obtained in Example 3 was analyzed. Cluster analysis was performed by the Ward's method with the Euclidean distance using the GeneSpring algorithm. For logistic regression analysis, statistical analysis free software "R" was used, and, MASS, caret, data.table was used as a library, and logistic regression analysis was performed.

Data 1: Regarding positive control reagent exposure data, cluster analysis was performed using GeneSpring to extract a group of genes sharing common fluctuation characteristics.

Data 2: Regarding the 3R4F reference cigarette exposure data, a group of genes that had commonly exhibited fluctuations during exposure to each tobacco smoke concentration was extracted.

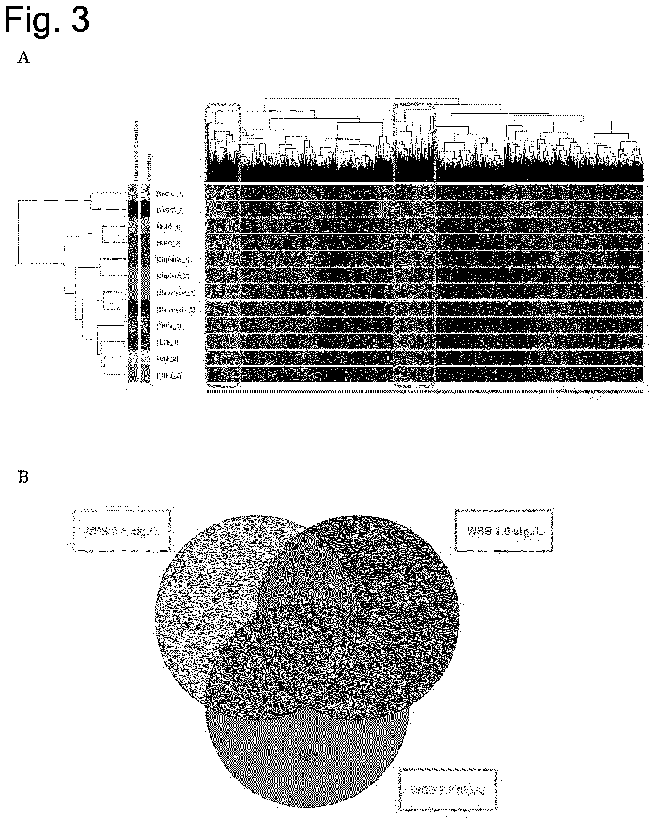

Through comparison of the gene groups extracted with the use of data 1: positive control reagent exposure data and data 2: 3R4F reference cigarette exposure data, 15 common genes (TXNIP, EFNA1, CYP1B1, ADM, AREG, DUSP6, SLC7A11, IGFBP3, WNT5A, CXCR4, PHLDA1, HILPDA, ZBED2, EGLN3, and FBXO32) were extracted (Fig. 3). Table 1 shows the results of examining the presence or the absence of previous reports on the functions of these genes and the association thereof with obstructive pulmonary disease.

[Table 1]

**Genes used for invention, and the in vitro evaluation for the risk of chronic obstructive pulmonary disease in the present functions thereof**

| Gene | Known functions of gene products |
| --- | --- |
| ADM | Prepo-hormone having multiple functions including vasodilation, hormone secretion, vascularization, and antimicrobial activity |
| AREG | EGF family member which interacts with EGF/TGF-$\alpha$ receptor to accelerate the normal growth of the above cells. |
| CXCR4 | CXC chemokine receptor specific to interstitial cell-derived factor-1 |
| CYP1B1 | Cytochrome P450 superfamily enzyme member. The high-level expression thereof is observed as a result of exposure to tobacco smoke. |
| DUSP6 | Bispecific protein of phosphatase subfamily. It negatively controls MAPK superfamily involved in cell proliferation and differentiation. |
| EFNA1 | Ephrin family member. Its target receptor contains protein tyrosine kinase and is involved in mediation of incident. |
| EGLN3 | Hypoxia inducible factor. It is essential for the control of hypoxia in neutrophil inflammation and plays an important role in DNA damage response. |
| FBXO32 | Fbox protein which functions in phosphorylation-dependent ubiquitination and the subsequent proteasomal degradation. |

(continued)

**Genes used for invention, and the in vitro evaluation for the risk of chronic obstructive pulmonary disease in the present functions thereof**

| Gene | Known functions of gene products |
|---|---|
| HILPDA | Hypoxia-inducible lipid droplet-associated protein. It stimulates cytokine expression, and accelerates cell growth and proliferation. |
| IGFBP3 | Insulin-like growth factor binding protein family. It prolongs the half-life of IGFs to alter the interaction with the cell surface receptors. |
| PHLDA1 | Each of proline-histidine rich proteins. It can play an important function in the anti-apoptotic effect of insulin-like growth factor-1. |
| SLC7A11 | Sodium-dependent, high-affinity antiporter which exchanges an anionic amino acid and anionic cysteine/glutamine with high specificity. |
| TXNIP | Thioredoxin binding protein which inhibits the antioxidative function of thioredoxin to cause active oxygen accumulation and cell stress. |
| WNT5A | Wnt family member 5A. It is a ligand for a member of the Frizzled family, 7-transmembrane receptors. |
| ZBED2 | Zinc finger BED domain-containing 2 |

Using the gene group of the above 15 genes, the microarray data (E-MTAB-1690, E-GEOD-20257, and E-MTAB-8545) of the lungs of healthy subjects and COPD patients (referred to as NS group and COPD group, respectively) included in the microarray database were subjected to discriminant analysis performed by logistic regression. The results are shown in Table 2.

[Table 2]

**Results of discriminating chronic obstructive pulmonary disease patients using 15 genes extracted in the present invention**

| Truth/Predicted | NS | COPD | True rate |
|---|---|---|---|
| **NS** | 33.8 | 7.1 | 0.826 |
| **COPD** | 7.6 | 51.5 | 0.871 |
| **Accuracy** | | 0.8531 | |

Results of cross-validation using the logistic regression model (5 -fold cross-validation was independently repeated 100 times). From the results of statistical analysis using the statistical analysis free software "R," the accuracy of discriminating the NS group from the other groups according to the model of the present invention was 0.826, the accuracy of discriminating the COPD group from the other groups was 0.871, and the overall accuracy was 0.8531, which were all extremely high accuracies. This means successful extraction of genes that enable COPD risk evaluation with the use of the in vitro 3D model of human airway epithelial cells.

Example 5 Risk evaluation by logistic regression analysis

In this Example, using the gene group of 15 genes found in Example 4 (TXNIP, EFNA1, CYP1B1, ADM, AREG, DUSP6, SLC7A11, IGFBP3, WNT5A, CXCR4, PHLDA1, HILPDA, ZBED2, EGLN3, and FBXO32), the public microarray data of actual healthy subjects, smokers, and COPD patients were subjected to discrimination performed by logistic regression analysis.

Backward elimination (method for calculating genes with high contribution based on AIC (Akaike information criterion) or BIC (Bayesian information criterion) was performed for searching the gene groups with the lowest AIC or BIC score. As a result, 11 genes (TXNIP, EFNA1, ADM, SLC7A11, IGFBP3, WNT5A, CXCR4, HILPDA, EGLN3, AREG, and FBXO32) were extracted as genes with high contribution to discrimination between healthy subjects and smokers, and 4 genes (EFNA1, TXNIP, DUSP6, and AREG) were extracted as genes with high contribution to discrimination between smokers and COPD patients. Table 3 below shows the results of the values (Estimate) of the intercepts and coefficients calculated by the logistic regression equation using genes after backward elimination, and their statistical values (Std. Error, Z value, Pr(>|Z|)).

[Table 3]

**Results of values of intercepts and coefficients calculated by the logistic regression equation using genes selected by backward elimination, and their statistical values**

| | Estimate | Std. Error | Z value | Pr(>|Z|) | | Estimate | Std. Error | Z value | Pr(>|Z|) |
|---|---|---|---|---|---|---|---|---|---|
| (Intercept) HLT\|SMK | 1.6715 | 0.4229 | 3.953 | 7.73E-05 | (Intercept) SMK\|COPD | -0.8555 | 0.2144 | -3.990 | 6.61E-05 |
| ADM | -2.2568 | 1.0247 | -2.202 | 0.027641 | AREG | -1.3039 | 0.7058 | -1.847 | 0.06469 |
| AREG | 2.0152 | 1.0407 | 1.936 | 0.05282 | DUSP6 | 1.4688 | 0.6254 | 2.349 | 0.01885 |
| CXCR4 | -3.1177 | 1.4308 | -2.179 | 0.029336 | EFNA1 | 2.3861 | 0.9058 | 2.634 | 0.00843 |
| EFNA1 | 3.7889 | 1.9572 | 1.936 | 0.052882 | TXNIP | -0.8847 | 0.5167 | -1.712 | 0.08687 |
| EGLN3 | -4.0571 | 1.7627 | -2.302 | 0.021357 | | | | | |
| FBXO32 | -3.8824 | 2.4113 | -1.610 | 0.107376 | | | | | |
| HILPDA | 3.2193 | 0.8100 | 3.974 | 7.06E-05 | | | | | |
| IGFBP3 | -8.2992 | 2.3747 | -3.495 | 0.000474 | | | | | |
| SLC7A11 | -3.5355 | 1.2516 | -2.825 | 0.00473 | | | | | |
| TXNIP | -5.6745 | 1.4851 | -3.281 | 1.33E-04 | | | | | |
| WNT5A | 2.7391 | 0.8231 | 3.328 | 8.75E-04 | | | | | |

| AIC | 105.3177 |
|---|---|
| BIC | 141.9099 |

| AIC | 166.2157 |
|---|---|
| BIC | 180.7789 |

The potential risk factor (PRF) was calculated from the probability calculated by each logistic regression equation. The onset probability and the potential risk factor were calculated as follows.

(i) The following probabilities were calculated on the basis of microarray data on the expression of 11 genes (TXNIP, EFNA1, ADM, SLC7A11, IGFBP3, WNT5A, CXCR4, HILPDA, EGLN3, AREG, and FBXO32) with use of intercepts and coefficients calculated in advance by logistic regression analysis using public data.

[Chemical Formula 10]

$$logit \ (P_{SMK}) = ln\left(\frac{P_{SMK}}{1 - P_{SMK}}\right) = C_{NS/SMK} + \beta_1 a_1 + \beta_2 a_2 + \cdots + \beta_{11} a_{11}$$

wherein $C_{NS|SMK}$ represents the intercept, $\beta_i$ represents the coefficient for each gene, and $a_i$ represents values after normalization of microarray data by GC-RMA; and the values of the intercepts and the coefficients are shown in Table 3.

(ii) On the basis of the microarray data on the expression of 4 genes (EFNA1, TXNIP, DUSP6, and AREG), the following probabilities were calculated using coefficients calculated in advance by logistic regression analysis using public data.

[Chemical Formula 11]

$$ logit(P_{COPD}) = ln\left(\frac{P_{COPD}}{1-P_{COPD}}\right) = C_{SMK/COPD} + \gamma_1 b_1 + \gamma_2 b_2 + \cdots + \gamma_5 b_5 $$

wherein $C_{SMK|COPD}$ represents the intercept, $\gamma_i$ represents the coefficient for each gene, and $b_i$ represents values after normalization of microarray data by GC-RMA; and the values of the intercepts and the coefficients are shown in Table 3. (iii) From the probability $P_{SMK}$ and the probability $P_{COPD}$, the risk of chronic obstructive pulmonary disease associated with inhalation of the cigarette smoke was calculated in terms of the following PRF.

[Chemical Formula 12]

$$ PRF = \frac{p_{SMK} \times p_{COPD}}{1-(p_{SMK} \times p_{COPD})} $$

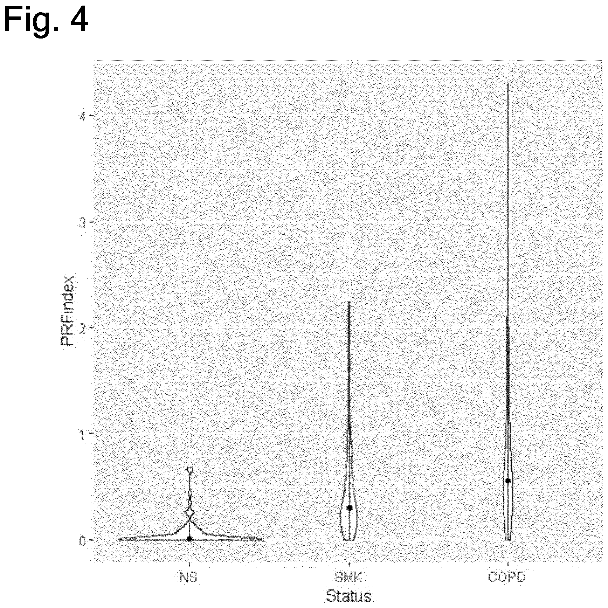

Using the above equation, the PRFs of healthy subjects (NS), healthy smokers (SMK), and COPD patients (COPD) were calculated. The results are shown in Fig. 4. As shown in Fig. 4, the PRF was 0.06 for non-smokers, 0.46 for smokers, and 0.83 for COPD patients, confirming an increase in PRF as compared to healthy non-smokers.

Example 6 Evaluation of risk of various concentrations and various exposure periods on flavor inhaler

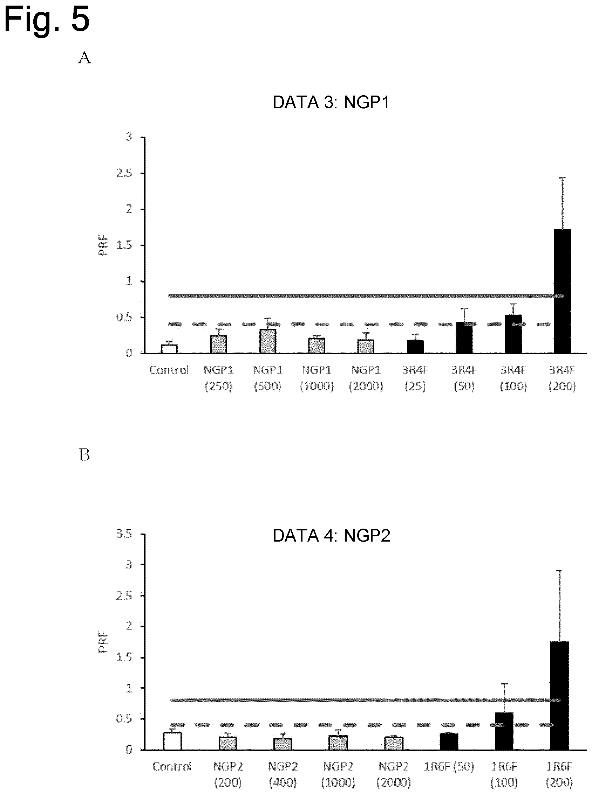

Using the regression equation described in Example 5, the PRFs in frequent exposure tests were calculated for various concentrations of various tobacco products and various exposure periods. Results are shown in Fig. 5. It was revealed that the average value of PRFs of the NGP exposure group was within the range set above at any concentration and any exposure period.

The risk of chronic obstructive pulmonary disease associated with smoking or inhalation was evaluated by the in vitro evaluation method, revealing that the PRF of NGP was much lower than that of existing combustible cigarettes.

INDUSTRIAL APPLICABILITY

The in vitro method for evaluating the risk of chronic obstructive pulmonary disease associated with smoking or inhalation of the present invention can be used for statistically quantitatively evaluating risk of chronic obstructive pulmonary disease associated with smoking or inhalation, which is conventionally-unknown. The in vitro method for evaluating the risk of chronic obstructive pulmonary disease associated with smoking or inhalation of the present invention can be used for research and development of products with reduced risks (products that may have reduced health risks associated with smoking or inhalation).

**Claims**

1. An in vitro method for evaluating the risk of chronic obstructive pulmonary disease associated with inhalation, comprising:

(1) calculating the following probability $P_{SMK}$ on the basis of data on the expression of 11 or more genes including at least the following 11 genes:
TXNIP, EFNA1, ADM, SLC7A11, IGFBP3, WNT5A, HILPDA, EGLN3, CXCR4, AREG, and FBXO32 from among the following 15 genes in airway epithelial cells exposed to a sample solution containing an aerosol or particulate matter in the aerosol from a heated tobacco product:
TXNIP, EFNA1, CYP1B1, ADM, AREG, DUSP6, SLC7A11, IGFBP3, WNT5A, CXCR4, PHLDA1, HILPDA, ZBED2, EGLN3, and FBXO32,
with use of an intercept and coefficients calculated in advance by logistic regression analysis using public data;

[Chemical Formula 1]

$$logit(P_{SMK}) = ln\left(\frac{P_{SMK}}{1 - P_{SMK}}\right) = C_{NS/SMK} + \beta_1 a_1 + \beta_2 a_2 + \cdots + \beta_x a_x$$

wherein $C_{NS|SMK}$ represents the intercept, $\beta_i$ represents the coefficient for each gene, $a_i$ represents gene expression data and represents a value after normalization of microarray data by GC-RMA or a quantitative value of gene expression obtained by a qPCR method, and x represents any integer of 11 to 15;
(2) calculating the following probability $P_{COPD}$ on the basis of data on the expression of 4 or more genes including at least the following 4 genes:
EFNA1, TXNIP, DUSP6, and AREG
from among the following 15 genes in airway epithelial cells exposed to a sample solution containing an aerosol or particulate matter in the aerosol from a heated tobacco product:
TXNIP, EFNA1, CYP1B1, ADM, AREG, DUSP6, SLC7A11, IGFBP3, WNT5A, CXCR4, PHLDA1, HILPDA, ZBED2, EGLN3, and FBXO32,
with use of an intercept and coefficients calculated in advance by logistic regression analysis using public data;

[Chemical Formula 2]

$$logit(P_{COPD}) = ln\left(\frac{P_{COPD}}{1 - P_{COPD}}\right) = C_{SMK/COPD} + \gamma_1 b_1 + \gamma_2 b_2 + \cdots + \gamma_y b_y$$

wherein $C_{SMK|COPD}$ represents the intercept, $\gamma_i$ represents the coefficient for each gene, $b_i$ represents gene expression data and represents a value after normalization of microarray data by GC-RMA or a quantitative value of gene expression obtained by the qPCR method, and y represents any integer of 4 to 10; and
(3) calculating the risk of chronic obstructive pulmonary disease associated with inhalation of the heated tobacco product, PRF, from the probability $P_{SMK}$ and the probability $P_{COPD}$.

[Chemical Formula 3]

$$PRF = \frac{p_{SMK} \times p_{COPD}}{1 - (p_{SMK} \times p_{COPD})}$$

2. The in vitro method according to claim 1, wherein in step (1), the probability $P_{SMK}$ is calculated by logistic regression analysis on the basis of data on the expression of all genes of 11 genes:
TXNIP, EFNA1, ADM, SLC7A11, IGFBP3, WNT5A, HILPDA, EGLN3, CXCR4, AREG, and FBXO32.

3. The in vitro method according to claim 1 or 2, wherein in step (2), the probability $P_{COPD}$ is calculated on the basis of data on the expression of all genes of 4 genes:
EFNA1, TXNIP, DUSP6, and AREG,
with use of coefficients calculated in advance by logistic regression analysis using public data.

4. The in vitro method according to any one of claims 1 to 3, wherein the airway epithelial cells are mammalian airway epithelial cells.

5. The in vitro method according to any one of claims 1 to 4, wherein the airway epithelial cells are human airway epithelial cells, mouse airway epithelial cells, or rat airway epithelial cells.

6. The in vitro method according to any one of claims 1 to 5, wherein the airway epithelial cells are three-dimensional cultured cells.

7. The in vitro method according to any one of claims 1 to 6, wherein the airway epithelial cells are under air-liquid interface culture.

8. The method according to any one of claims 1 to 7, wherein when the PRF in the airway epithelial cells exposed to a solution containing an aerosol or particulate matter in the aerosol from a heated tobacco product at a maximum concentration of 2000 puffs/L for 24 hours is in the range of 0.8 or less, it is determined that the risk of chronic obstructive pulmonary disease associated with the inhalation of the heated tobacco product is low or absent.

9. The method according to any one of claims 1 to 8, wherein the PRFs of two types of heated tobacco products are compared, and it is determined that the one with lower PRF has the risk of chronic obstructive pulmonary disease associated with inhalation lower than that of the other.

10. Use of

(1) data on the expression of 11 or more genes including at least the following 11 genes:
TXNIP, EFNA1, ADM, SLC7A11, IGFBP3, WNT5A, HILPDA, EGLN3, CXCR4, AREG, and FBXO32 from among the following 15 genes:
TXNIP, EFNA1, CYP1B1, ADM, AREG, DUSP6, SLC7A11, IGFBP3, WNT5A, CXCR4, PHLDA1, HILPDA, ZBED2, EGLN3, and FBXO32, and
(2) data on the expression of 4 or more genes including at least the following 4 genes:
EFNA1, TXNIP, DUSP6, and AREG
from among the following 15 genes:
TXNIP, EFNA1, CYP1B1, ADM, AREG, DUSP6, SLC7A11, IGFBP3, WNT5A, CXCR4, PHLDA1, HILPDA, ZBED2, EGLN3, and FBXO32,
for an in vitro method for evaluating the risk of chronic obstructive pulmonary disease associated with inhalation.

11. A heated tobacco product having a structural feature of electrically heating directly or indirectly an aerosol source comprising reconstituted tobacco, wherein
when the in vitro method according to any one of claims 1 to 6 is performed using a sample solution containing an aerosol or particulate matter in the aerosol from the heated tobacco product, PRF is in the range of 0.8 or less in airway epithelial cells exposed to the solution containing an aerosol or the particulate matter in the aerosol from the heated tobacco product at a maximum concentration of 2000 puffs/L for 24 hours.

12. The heated tobacco product according to claim 11, wherein the reconstituted tobacco is in the form of reconstituted tobacco granules having an average particle size of 0.2 mm or less, and
the reconstituted tobacco granules are heated by a mixed vapor of propylene glycol and glycerin produced by heating all or part of a carrier comprising a porous substance or a fiber with an electric heater.

13. The heated tobacco product according to claim 11, wherein the reconstituted tobacco is a reconstituted tobacco sheet having an average width of 1 mm or more and an average length of 5 mm or less, and is wrapped in rolling paper to form a tobacco rod, and
the tobacco rod is in contact with the circumference of the rolling paper, and thus is heated by an electric heater.

14. The heated tobacco product according to any one of claims 11 to 13, wherein an aerosol produced contains acetaldehyde in an amount of 1.4 $\mu$g/rod or less.

15. The heated tobacco product according to any one of claims 11 to 13, wherein an aerosol produced contains acrolein in an amount of 1.2 $\mu$g/rod or less.

16. The heated tobacco product according to any one of claims 11 to 13, wherein an aerosol produced contains formaldehyde in an amount of 68.4 $\mu$g/rod or less.

## Fig. 1

## Fig. 2

# Fig. 3

A

B

Fig. 4

# Fig. 5

A

DATA 3: NGP1

B

DATA 4: NGP2

<table>
<tr><td colspan="2" align="center">**INTERNATIONAL SEARCH REPORT**</td><td colspan="2">International application No.<br>PCT/JP2019/049086</td></tr>
</table>

**A. CLASSIFICATION OF SUBJECT MATTER**
Int. Cl. A24F47/00(2020.01)i, C12Q1/06(2006.01)i, C12Q1/6837(2018.01)i,
C12Q1/6851(2018.01)i, C12Q1/686(2018.01)i, C01N33/50(2006.01)i
FI: C12Q1/6837 Z, G01N33/50 P, C12Q1/6851 Z, C12Q1/686 Z, C12Q1/06, A24F47/00

According to International Patent Classification (IPC) or to both national classification and IPC

**B. FIELDS SEARCHED**

Minimum documentation searched (classification system followed by classification symbols)
Int. Cl. A24F47/00, C12Q1/06, C12Q1/6837, C12Q1/6851, C12Q1/686,
G01N33/50

Documentation searched other than minimum documentation to the extent that such documents are included in the fields searched
Published examined utility model applications of Japan     1922-1996
Published unexamined utility model applications of Japan   1971-2020
Registered utility model specifications of Japan           1996-2020
Published registered utility model applications of Japan   1994-2020

Electronic data base consulted during the international search (name of data base and, where practicable, search terms used)
WPI; JSTPlus/JMEDPlus/JST7580 (JDreamIII);CAplus/MEDLINE/EMBASE/BIOSIS/WPIDS
(STN);PubMed

**C. DOCUMENTS CONSIDERED TO BE RELEVANT**

| Category* | Citation of document, with indication, where appropriate, of the relevant passages | Relevant to claim No. |
|---|---|---|
| X | JP 2016-528234 A (VOERMAN, Gerard) 15 September 2016, claims 1, 12, 14, 15, paragraph [0044] | 11–13 |
| Y | | 14–16 |
| X | WO 2018/045174 A1 (BAYBUTT, RICHARD) 08 March 2018, claims 1, 12, 19, 30 | 11–13 |
| Y | | 14–16 |
| Y | JP 2018-78902 A (ALTRIA CLIENT SERVICES LLC) 24 May 2018, claims, paragraph [0064] | 14–16 |
| Y | JP 2018-78805 A (TRADE WORKS CO., LTD.) 24 May 2018, claim 1, paragraph [0014], examples | 14–16 |

☒ Further documents are listed in the continuation of Box C.     ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>21.02.2020 | Date of mailing of the international search report<br>03.03.2020 |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.

PCT/JP2019/049086

C (Continuation). DOCUMENTS CONSIDERED TO BE RELEVANT

| Category* | Citation of document, with indication, where appropriate, of the relevant passages | Relevant to claim No. |
|---|---|---|
| A | JP 2017-519521 A (TRANSGENION-INTERNATIONAL INSTITUTE FOR REGENERATIVE TRANSLATIONAL MEDICINE GMBH) 20 July 2017, entire text, all drawings | 1-16 |
| A | US 2006/0211026 A1 (BELLONI, P. N. et al.) 21 September 2006, entire text, all drawings | 1-16 |
| A | US 2014/0135302 A1 (SOMALOGIC, INC.) 15 May 2014, entire text, all drawings | 1-16 |
| A | US 2016/0024583 A1 (ALLEGRO DIAGNOSTICS CORP.) 28 January 2016, entire text, all drawings | 1-16 |
| A | WO 2006/105252 A2 (THE REGENTS OF THE UNIVERSITY OF COLORADO) 05 October 2006, entire text, all drawings | 1-16 |
| A | US 2010/0119474 A1 (CRYSTAL, R. G. et al.) 13 May 2010, entire text, all drawings | 1-16 |
| A | WO 2013/190092 A1 (PHILIP MORRIS PRODUCTS S.A.) 27 December 2013, entire text, all drawings | 1-16 |
| A | STEILING, K. et al., A Dynamic Bronchial Airway Gene Expression Signature of Chronic Obstructive Pulmonary Disease and Lung Function Impairment, AMERICAN JOURNAL OF RESPIRATORY AND CRITICAL CARE MEDICINE, 01 May 2013., vol. 187, iss. 9, pp. 933-942, entire text, all drawings | 1-16 |
| A | GOSSELINK, J. V. et al., Differential Expression of Tissue Repair Genes in the Pathogenesis of Chronic Obstructive Pulmonary Disease, AMERICAN JOURNAL OF RESPIRATORY AND CRITICAL CARE MEDICINE, 14 January 2010, vol. 181, pp. 1329-1335, entire text, all drawings | 1-16 |
| A | SAVARIMUTHU FRANCIS, S. M. et al., Genes and Gene Ontologies Common to Airflow Obstruction and Emphysema in the Lungs of Patients with COPD, PLOS one, 15 March 2011, vol. 6, Issue 3, e17442, pp. 1-7, entire text, all drawings | 1-16 |

Form PCT/ISA/210 (continuation of second sheet) (January 2015)

**INTERNATIONAL SEARCH REPORT**

| International application No. |
| --- |
| PCT/JP2019/049086 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | BHATTACHARYA, S. et al., Molecular Biomarkers for Quantitative and Discrete COPD Phenotypes, AMERICAN JOURNAL OF RESPIRATORY CELL AND MOLECULAR BIOLOGY, 10 October 2008, vol. 40, pp. 359-367, entire text, all drawings | 1-16 |
| A | JP 2004-121218 A (JENOKKUSU SOYAKU KENKYUSHO KK) 22 April 2004, entire text, all drawings | 1-16 |

Form PCT/ISA/210 (continuation of second sheet) (January 2015)

**INTERNATIONAL SEARCH REPORT**
Information on patent family members

| | | International application No. |
|---|---|---|
| | | PCT/JP2019/049086 |

| Patent Documents referred to in the Report | Publication Date | Patent Family | Publication Date |
|---|---|---|---|
| JP 2016-528234 A | 15.09.2016 | US 2016/0177285 A1<br>claims 1, 12, 14, 15,<br>paragraph [0051]<br>WO 2016/020070 A1<br>EP 3030254 A1<br>CA 2920452 A<br>AU 2014304578 A<br>CN 105592854 A<br>KR 10-2016-0054475 A<br>EA 201690335 A | |
| WO 2018/045174 A1 | 08.03.2018 | US 2019/0192449 A1 | |
| JP 2018-78902 A | 24.05.2018 | US 2013/0192615 A1<br>claims, paragraph<br>[0083]<br>WO 2013/116558 A1<br>EP 2809180 A1<br>AU 2013214987 A<br>CA 2862105 A<br>IL 233851 A<br>CA 2867620 A<br>KR 10-2014-0125822 A<br>CN 104219973 A<br>MX 2014009396 A<br>MA 20150056 A<br>NZ 628058 A<br>RU 2014135386 A<br>UA 111988 C<br>ES 2612531 T<br>PL 2809186 T<br>BR 112014018881 A<br>PL 2809182 T | |
| JP 2018-78805 A | 24.05.2018 | (Family: none) | |
| JP 2017-519521 A | 20.07.2017 | US 2017/0349947 A1<br>entire text, all<br>drawings<br>WO 2015/185653 A2<br>EP 3152328 A1<br>CA 2953209 A | |
| US 2006/0211026 A1 | 21.09.2006 | WO 2006/097244 A2 | |
| US 2014/0135302 A1 | 15.05.2014 | WO 2014/074682 A1<br>CN 104812913 A<br>HK 1211991 A | |
| US 2016/0024583 A1 | 28.01.2016 | WO 2014/186036 A1<br>EP 2968988 A1 | |
| WO 2006/105252 A2 | 05.10.2006 | (Family: none) | |
| US 2010/0119474 A1 | 13.05.2010 | WO 2008/109773 A2 | |
| WO 2013/190092 A1 | 27.12.2013 | (Family: none) | |
| JP 2004-121218 A | 22.04.2004 | US 2005/0208496 A1<br>entire text, all<br>drawings<br>EP 1394274 A2 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013190092 A
- WO 2016075748 A
- WO 2016075749 A
- JP 2018213498 A

**Non-patent literature cited in the description**

- *Am. J. Respir Crit Care Med.,* 2007, vol. 175 (6), 577-586